# EUROPEAN PATENT SPECIFICATION

(11) **EP 1 951 074 B1**
(45) Date of publication and mention of the grant of the patent: **02.07.2014**
(21) Application number: 06812981.6
(22) Date of filing: 07.11.2006
(51) Int. Cl.: A24B 15/18, A24B 15/24, A24B 3/18, A24B 15/12

(54) **METHOD FOR PRODUCING NICOTINE WITH A REDUCED CONTENT OF NITROSAMINES**
VERFAHREN FÜR DIE HERSTELLUNG VON NIKOTIN MIT REDUZIERTEM GEHALT AN NITROSAMINEN
PROCEDE POUR LA FABRICATION DE NICOTINE A TENEUR REDUITE EN NITROSAMINES

(30) Priority: 07.11.2005 SE 0502462; 10.11.2005 US 735305 P
(43) Date of publication of application: 06.08.2008
(73) Proprietor: Swedish Match North Europe AB, 118 85 Stockholm (SE)
(72) Inventor: ERICSSON, Thomas, S-260 41 Nyhamnsläge (SE); BÜLTROPP LUNELL, Lars, Erik, S-252 84 Helsingborg (SE); GOVINDJI VIRJI, Mihir, Mumbai-400020 (IN)
(74) Representative: Nikolopoulou, Sofia
(86) International application number: PCT/SE2006/001258
(87) International publication number: WO 2007/053097

(56) References cited:
- WO-A1-02/28209
- WO-A1-02/087365
- WO-A1-2004/098323
- WO-A1-2005/122803
- WO-A2-2004/068973
- US-A1- 2004 025 891
- WARFIELD A H: "Research proposal: technology for tsna removal from tobacco extracts", INTERNET CITATION, 11 September 1987 (1987-09-11), pages 1-6, XP002539652, Retrieved from the Internet: URL:http://legacy.library.ucsf.edu:8080/q/ l/o/qlo68e00/Sqlo68e00.pdf [retrieved on 2009-07-31]

## Description

### FIELD OF INVENTION

The invention relates to methods producing nicotine comprising less than 0.1 ug nitrosamines/ mg nicotine.

### BACKGROUND OF INVENTION

Smoking articles, (e. g., cigarettes, cigars, pipes, etc) and smokeless tobacco products (e. g., chewing tobacco, dry and moist snuff, etc.) are made from natural tobacco, reconstituted tobacco, and blends thereof. Natural tobacco may contain carcinogenic nitrosamines as ell as other harmful components, such as Pb, Hg, Cd etc. Such components are formed during the curing of tobacco, e.g., tobacco-specific nitrosamines (TSNAs) and non-tobacco specific nitrosamines. Likewise, reconstituted tobacco formed from natural tobacco by-products may also contain nitrosamines. The nitrosamines may be transferred from tobacco products to man when used in snuff, chewing tobacco or other products made thereof. In addition, the smoke produced by tobacco products containing nitrosamines can also contain nitrosamines, which are either transferred from tobacco or pyro-synthesized. These substances have been shown to be harmful to man, mainly due to their carcinogenic properties. Extensive research has been conducted on the removal of nitrosamines and TSNAs in tobacco products.

Despite such attempts to remove TSNAs from tobacco, a need currently exists for an improved method of reducing the content of nitrosamines (e. g., TSNAs) in tobacco.

Smokeless tobacco (SLT) has been associated with oral cancer for many decades. The epidemiologic studies addressing the human risks of SLT use consists primarily of case-control studies performed over a 40-year period starting in 1957. Some of the compounds may promote cancer development in long-term SLT users.

WO 02/28209 discloses a method in which the content of the nitrosamines are reduced from 0,84 microgram/g dry tobacco to 40 nanograms per gram tobacco in solution, i.e., not dry tobacco (see example 3) or from 4 microgram /g dry tobacco to 0,8 microgram /gram (see example 1). Activated charcoal or zeolites was used to reduce the amount of the nitrosamines.

EP 1 623 634 A1 discloses a process for producing regenerated tobacco, wherein a tobacco material is extracted in a solvent, whereupon the solvent is fractionated and desired components are transferred back to the tobacco material for the production of the regenerated tobacco material. One aim of the invention disclosed is to reduce the amount of TSNAs in the regenerated product.

EP 1 782 702 A1 likewise discloses a process for producing regenerated tobacco, wherein nicotine and TSNAs are separated by way of extraction using e.g. activated carbon and an aqueous extraction solvent.

WO 2004/068 973 A2 discloses treatment of tobacco leaves by spraying or soil treatment. Treatment of growing plants is made with a chemical solution commonly containing a plant growth hormone, a plant activator, a herbicide and a stress inducing agent. The treatment has been shown to stimulate the production of antioxidants in the tobacco leaves, which interferes with the production of TSNAs.

WO 02/087365 discloses a smokeless product suitable for human consumption in which said product comprises a content of the nitrosamines which are 0,3 ug /g tobacco or less. The tobacco is prepared from tobacco plants having a low content of TSNA, i.e., the variety Virginia flue (see page 6).

Therefore there is a need of developing new methods that enables the possibility to produce nicotine with a reduced amount of carcinogenic components and thereby be able to produce new nicotine as well as new tobacco products with less amounts of the carcinogenic components, such as the nitrosamines as well as the heavy metals.

### BRIEF DISCLOSURE OF THE INVENTION

The invention relates to a novel process as well as nicotine and nicotine products produced by the method according to claim 1 and products comprising said nicotine wherein said nicotine has a reduced level of at least nitrosamines. The nicotine comprises less than 0.1 ug nitrosamines/ mg nicotine, which are harmful for the human being consuming said nicotine or give a bad taste such as a bitter taste to the nicotine product.

The invention also relates to a method how to obtain nicotine wherein said method comprising the steps of; providing tobacco material comprising nicotine and fibres, separating said fibres from said tobacco material, extracting nicotine from said tobacco material, obtaining nicotine, wherein said nicotine comprises less than 0.1 ug nitrosamines/mg nicotine.

By a unique combination of how to grow the tobacco plants, harvesting of the plant material as well as the purification process it is for the first time possible to provide a purer nicotine having reduced amount of nitrosamines, which are harmful for the human being as well as giving the nicotine product a bitter taste and reducing the coloration upon use as a nicotine or tobacco product, such as Swedish snus.

### DETAILED DESCRIPTION OF THE INVENTION

### Definitions

In the context of the present application and invention, the following definitions apply:
The term " tobacco" refers to any part of a tobacco plant or mixtures of tobacco plants arising from the same or different species as well as being genetically modified (GMO). The tobacco plant belongs to the family Solanaceae and the genus Nicotiana. Examples of tobacco plants are those from *Nicotiana tabacum* and *Nicotiana rustica.* Examples of parts are leaves, stems, roots etc, which may be obtained by any method such as being fresh, frozen, deep-frozen, freeze dried or cured.
The term "nicotine product" refers to any product such as inhaler, tablet, capsule, lozenge, chewing gum, mucosal or transdermal patch, nasal drops, nasal and/or oral spray, transdermal gel/cream or any other topical administration, rectal application, etc, used in any application where nicotine is to be delivered to the human body.
The term "tobacco product" refers to any product made from or containing tobacco such as nasal snuff, oral moist or dry snuff, chewing tobacco, pipe tobacco, cigar or cigarette tobacco, tablet, capsule, lozenge, chewing gum, mucosal or transdermal patch etc, used in any application where nicotine is to be delivered to the human body. It also refers to any such nicotine-free product.
The term "tobacco specific nitrosamines" (TSNA) refers to a number of nitrosamines, such as N'-nitrosonomicotine (NNN), N'-nitrosoanatabine (NAT), N'-nitrosoanabasine (NAB), and 4-(N-methylnitrosamino)-1-(3-pyridyl)-1-butanone (NNK), N'-nitrosonomicotine.
The term "tobacco fibre" is intended to mean any part of the tobacco plant comprising cellulose, cellulose fibres and/or starch.

### Nicotine

The invention relates to a method of providing nicotine having improved properties compared to other nicotines, such as a low content of the nitrosamines, such as having a content of the nitrosamines being less than 0.1 ug nitrosamines/mg nicotine, such as less than 0.09, 0,08, 0,07, 0,06, 0,05, 0,04, 0,03, 0,02, 0,01 ug/mg nicotine or even less than 0.01ug nitrosamines/mg nicotine. Examples are 0.009, 0.008, 0.007, 0.006, 0.005, 0.004, 0.003, 0.002, or 0.01, 0.009, 0.008, 0.007, 0.006, 0.005, 0.004, 0.003, 0.002 or 0.001 ug nitrosamines/mg nicotine or even lower, i.e., the nicotine produced may be substantially free from nitrosamines. The nitrosamines may be N'-nitrosonornicotine (NNN), N'-nitrosoanatabine (NAT), N'-nitrosoanabasine (NAB), and 4-(N-methylnitrosamino)-1-(3-pyridyl)-1-butanone (NNK), and 4-(Methylnitrosamino)-1-(3-pyridyl)-1-butanone.

A reduction of the above mentioned compounds will, give rise to a healthier nicotine and nicotine product, since these products are carcinogenic or otherwise harmful to the body or giving the nicotine product a bitter taste. The nicotine may be substantially free from tobacco fibres, i.e., the insoluble fibres. The nicotine produced may be stabilised by the formation of a salt such as with an organic acid. Examples of acids are sulphuric acid.

The nicotine obtained may be used to produce nicotine product as well as tobacco products. The nicotine product comprises above defined nicotine as well as an acceptable diluent, buffer, carrier or excipient, such as a pharmaceutically acceptable ones. "Acceptable" means a diluent, buffer, carrier or excipient that at the dosage and concentrations employed does not cause any unwanted effects in patients. Such acceptable buffers, carriers or excipients are well-known in the art (see for example Remington's Pharmaceutical Sciences, 18th edition, A.R Gennaro, Ed., Mack Publishing Company (1990) and handbook of Pharmaceutical Excipients, 3rd edition, A. Kibbe, Ed., ., Pharmaceutical Press (2000). Examples of carriers include carriers selected from the group consisting of ion exchange resins of cross linked polyacrylic, styrene, styrene/DVB, cross linked polymethacrylic, polacrilex, , cyclodextrin, dextrin, sugar alcohols, polyethylene glycol, starch, microcrystalline cellulose, cellulose and cellulose derivatives, chewing gum bases, gelatine, alginates, polyvinylpyrrolidone or derivates thereof, natural or modified fibres and fibres, such as tobacco fibres.

The nicotine in the nicotine product may be admixed with adjuvants such as lactose, sucrose, sugar alcohols, starch powder, microcrystalline cellulose, polyethylene glycol, microcrystalline cellulose, cellulose and cellulose derivatives cellulose esters of alkanoic acids, stearic acid, talc, cyclodextrin, dextrin, magnesium stearate, magnesium oxide, sodium and calcium salts of phosphoric and sulphuric acids, acacia, gelatine, alginates, polyvinylpyrrolidone, and/or polyvinyl alcohol, and tabletted or encapsulated for conventional administration.

Alternatively, nicotine may be dissolved in saline, water, polyethylene glycol, propylene glycol, ethanol, oils, tragacanth gum, and/or various buffers. Other adjuvants and modes of administration are well known in the pharmaceutical art. The carrier or diluent may include controlled release material, such as glyceryl monostearate or glyceryl distearate alone or with a wax, or other materials well known in the art.

The nicotine product may be subjected to conventional pharmaceutical operations such as sterilization and/or may contain conventional adjuvants such as preservatives, stabilization, wetting agents, emulsifiers, buffers, fillers, etc., e.g., as disclosed elsewhere herein.

The nicotine product directly obtained from the method may be administered locally or systemically, such as topically, intravenously, orally, and even rectal use is possible.

Examples of nicotine products are tablets, capsules, lozenges, chewing gums, transmucosal or transdermal patches, nasal drops, nasal or oral spray. Other examples are smoking articles, such as cigarettes, cigars, fine cut smoking articles and non-smoking articles, such as nasal snuff, oral moist or dry snuff, and chewing tobacco.

Additionally the nicotine may be used to produce a tobacco product, such as a tobacco product comprising tobacco nicotine as defmed above. The nicotine product or tobacco product may also comprise one or more additives selected from the group consisting of natural, natural identical, synthetic and semisynthetic additives such as menthol, bergamot, eucalyptus and coffee, orange, mandarin, acai berry,citrus or other fruit flavour, liquorice, etc. Further, the nicotine product or tobacco product may also comprise monosaccharides such as glucose, fructose, sorbitol, Xylitol maltitol, mannitol, or sweeteners such as saccharin, sodium saccharin, cyclamates, Acesulfan K aspartam. Moreover, the nicotine or tobacco product may also comprise polysaccharides such as sucrose, starch, maltodextrin, natural fibres, cellulose, starches or other suitable fillers, Avicel; Microcrystalline cellulose, talc and natural and nature identical aromas and flavours. A typical formulation for 3 mg snus portion, Tobacco 10 to 24 %, Fibre 10 to 20 %, Polydextrose 5 to 15%, Sodium Chloride 8 %, Sodium bicarbonate 7 %, polyvinyl pyrrolidon 3 %, Talc 2 %, Avicel 5 to 15 %, flavours quantum sati. Vad betyder detta ordet?

The tobacco product may have the tobacco fibre material as a core and the tobacco nicotine and/or salt adsorbed onto the tobacco fibre material or absorbed into the tobacco fibre material, such as by spraying the nicotine and/or salt onto the tobacco fibre material. Additionally, the nicotine and/or the fibres from tobacco may be encapsulated into non-woven materials, well-known for a person skilled in the art.

The nicotine product or the tobacco product may be formulated into one or more preparations, such as sachets, pouches, stick-pack, buccal pads or patches or compressed into chewing tablets, lozenges, resoriblettes or tablets for sucking. The resulting product is then packed in air-tight packages to prevent losses by oxidization and/or vaporization of the nicotine.

The nicotine product or tobacco product produced according to the invention may have a release profile *in vitro* analysed during 35 minutes, with following intervals;
Nicotine released after 5 minutes is 0,1, 0,2, 0,3, 0,4, 0,5, 0,6, 0,7, 0,8, 0,9, 1, 2, 3, 4, 5, 6, 7, 8, 9, 10 mg.
Nicotine released after 10 minutes is 0,1, 0,2, 0,3, 0,4, 0,5, 0,6, 0,7, 0,8, 0,9, 1, 2, 3, 4, 5, 6, 7, 8, 9, 10 mg.
Nicotine released after 15 minutes is 0,1, 0,2, 0,3, 0,4, 0,5, 0,6, 0,7, 0,8, 0,9, 1, 2, 3, 4, 5, 6, 7, 8, 9, 10 mg.
Nicotine released after 20 minutes is 0,1, 0,2, 0,3, 0,4, 0,5, 0,6, 0,7, 0,8, 0,9, 1, 2, 3, 4, 5, 6, 7, 8, 9, 10 mg.

Nicotine released after 25 minutes is 0,1, 0,2, 0,3, 0,4, 0,5, 0,6, 0,7, 0,8, 0,9, 1, 2, 3, 4, 5, 6, 7, 8, 9, 10 mg.
Nicotine released after 30 minutes is 0,1, 0,2, 0,3, 0,4, 0,5, 0,6, 0,7, 0,8, 0,9, 1, 2, 3, 4, 5, 6, 7, 8, 9, 10 mg.
Nicotine released after 35 minutes is 0,1, 0,2, 0,3, 0,4, 0,5, 0,6, 0,7, 0,8, 0,9, 1, 2, 3, 4, 5, 6, 7, 8, 9, 10 mg.

The release profile may be designed with respect to the rate and amount of nicotine and unwanted substances released by the formulation. The release of nicotine from the nicotine or tobacco product may be designed to be released in a sufficient amount and/or a sufficient rate to enable a desired effect and on the other hand be designed to retain substances, which normally give rise to side-effects, such as, e. g., nitrosamines, and/or to release such substances in a substantially low amount and/or at a substantially slow rate.

In another embodiment of the invention the release of the nicotine obtained according to the claimed method, may be delayed compared to the commercially available moist snuff, to mimic the effect of nicotine replacement products such as, e.g., patches. The release profile may be 0.1-10 mg/h.

The tobacco products may be designed so that at least about 50% w/w of the total content of nicotine is released within at the most 60 min when subj ect to the release test described in the European pharmacopoeia latest ed.

The tobacco product obtained according to the claimed method may be designed so that at least about 50% w/w of the total content of nicotine is released within at the most 120 min when subject to the release test.

### Extracted release

Normally release is tested in in-vitro studies, extracted release is the amount release during use of a tobacco product such as snuff, snus or a nicotine product. Mean extracted amount is calculated by subtraction of individual or mean values of used product from the mean content of ten "unused products".

Whether the nicotine steady state or single plasma levels from a refined nicotine or tobacco product based on natural tobacco and/or genetically modified and/or a tobacco substitute and/or nicotine according to the invention, the tobacco and/or tobacco substitute and/or pharmaceutical product may preferably have the calculated extracted amount in humans as typically; extraction (mg) 0,1, 0,2, 0,3, 0,4, 0,5, 0,6, 0,7, 0,8, 0,9, 1, 2, 3, 4, 5, 6, 7, 8, 9, 10 mg. Whether the nicotine steady state or single plasma levels from a refined tobacco product based on natural tobacco and/or genetically modified and/or a tobacco substitute and/or nicotine according to the invention, the tobacco and/or tobacco substitute and/or pharmaceutical product may preferably have the biological parameters in humans as typically;
Cmax (ng/ml) 1, 2, 3, 4, 5, 6, 7, 8, 9, 10, 11, 12, 13, 14, 15, 16, 17, 18, 19, 20, 21,22, 23, 24, 25, 26, 27, 28, 29, 30, 32, 33, 34, 35, 36, 37, 3 8, 39, 40 or more up to 50 ng/ml. AUC (hours x ng/ml) 1, 2, 3, 4, 5, 6, 7, 8, 9, 10, 11, 12, 13, 14, 15, 16, 17, 18, 19, 20, 21,22, 23, 24, 25, 26, 27, 28, 29, 30, 32, 33, 34, 35, 36, 37, 38, 39, 40 or more up to 50 hours x ng/ml.

Whether the nicotine pH levels from a nicotine or a tobacco product according to the invention may have the pH parameters as typically;
pH 7,0, 7,1, 7,2, 7,3, 7,4, 7,5, 7,6 7,8, 7,9, 8,0, 8,1, 8,2, 8,3, 8,4, 8,5, 8,6 8,8, 8,9, 9,0, 9,1 to 10.

The nicotine is produced by the claimed method, as described in the examples.

### A method of producing said tobacco product

The invention also relates to a method of producing nicotine and a tobacco product as described above. Tobacco cuttings are planted in the fields as well as seeds in which the soil has a low content of heavy metals to secure that heavy metals will not be accumulated in the tobacco plants and later on be present in the nicotine after the nicotine has been purified from the tobacco plants. The nicotine is extracted from tobacco, such as from *Tobacco tabacum.* The tobacco plant may be a genetic modified plant, such as a genetic modified plant in which the content of the nitrosamines have been reduced. The tobacco plant material may be any part of the plant such as roots, stems or leaves such as lamina of the leaf. The tobacco plants may be grown on farmland or in greenhouse, with or without fertilizers or addition of other growth stimulating components. The choice of fertilizer depends on the quality of the soil in the field and well-known for a person skilled in the art. Examples of fertilizers are organic fertilizers, NPK or Urea. In a specific embodiment the plant material is lamina, which is harvested and directly transferred into a temperature being below 0°C, such as being frozen, by freezing in liquid nitrogen and/or dry ice and/or deep freezing containers and/or any other technique, which halt all the ongoing processes within the plant and thereby reduce and/eliminate the possibility that nitrosamines will be expressed to elevated amounts within the plant material. Alternatively the tobacco plant material is green material without any visible yellow parts. By securing that the plant material is not stressed the levels of the nitrosamines will be kept lower as compared to when the plants are under any pressure.

The harvested plant material may then be crushed, cut, milled or sliced and mixed with a water solution to which a base is added such as sodium hydroxide (NaOH) or calcium carbonate (CaCO3, limestone) or calcium hydroxide (slaked lime) 10 % by weight and 25% moisture. The plant mixture may then be packed into a vessel, such as a percolator or a reactor vessel and water may then be passed through the percolator to enable the nicotine to be extracted from the plant material. The fibres are then removed from the nicotinized broth and the nicotinized broth is further processed to purify the nicotine therein.

The nicotinized broth may be added to a cationic exchange resin, such as Amberlite™, such as Amberlite™ IPR 64, or Sepharose Fast Flow, (both may be obtained from Sigma-Aldrich) or Indion 464 supplied by Ion Exchange (India) Ltd., passed through the column whereby the nicotine will be bound to the column. The nicotine may then be released from the column by the use of an acid such as by the use of acetic or phosphoric acid. After such method the nicotine will be released/extracted from the plant fibres. By the use of a cationic column the nicotine that will be obtained after passing said column will be purer as compared to not using a cationic column.

The method gives rise to the opportunity to separate the nicotine and the fibre material from the Tobacco plant material and removing and/or minimising the amount of certain compounds within the nicotine as well as within the fibre material, such as carcinogenic as well as bitter tasting components. The plant material may be exposed to a subset of washing steps such as both alkaline and acid washing steps to remove unsuitable components on or within the material. Examples of washing steps are washing with NaOH and water or purified water.

The washing steps may be performed one or several times depending on the starting material. Samples may be taken after every washing step to follow the washing effect on the unwanted components, which are reduced and/or eliminated by the washing steps. The numbers of washing steps are dependent on the tobacco plant material and the number of washing steps may vary from time to time. However, a person skilled in the art will know how many washing steps that are needed to obtain the invented tobacco product by using the different tests that are shown in the examples.

The numbers of washing steps are dependent on the tobacco plant material and the number of washing steps may vary from time to time. However, a person skilled in the art will know how many washing steps that are needed to obtain the invented tobacco product by using the different tests that are shown in the examples.

The nicotine obtained by the invented method having the same characteristics as the above defined nicotine.

Following examples are intended to illustrate, but not to limit, the invention in any manner, shape, or form, either explicitly or implicitly.

### EXAMPLES

### EXAMPLE 1

### PURIFICATION OF NICOTINE FROM DEEP FROZEN TOBACCO LEAVES USING A CATION EXCHANGE RESIN.

### Stage 1 Harvesting and deep freezing

Fresh tobacco leaves from tobacco plants (="green tobacco") are collected and deep frozen (-20°C) within less than 3 hours.

### Stage II Mixing and Percolation

The deep frozen tobacco leaves and calcium carbonate (CaCO3, limestone) or calcium hydroxide (slaked lime) 10 % by weight are mixed in a Mixing machine (ribbon) of Machine Steel Water is added to bring the moisture of the mixture to 25 % humidity. The slightly humid raw tobacco mixture is packed into a Machine Steel percolator, i.e. a cylindrical extractor. Purified water is percolated through the mixture to get a nicotinized broth.

### Stage III Extraction

This nicotinized broth, i.e. the nicotine containing aqueous mother-liquor is transferred to a moderate-strong acid cation exchange resin, i.e., Amberlite™ IPR64 (Rohm and Haas/Ion Exchange Resins, Philadelphia, PA, USA or Indion 464 supplied by Ion Exchange (India) Ltd Tiecicon House, Dr. E. Moses Road, Mahalaxmi, Mumbai-400 011, India) packed in tall columns of Machine Steel or Stainless Steel. The nicotine - nicotine cation exchange resin complex is washed with purified water to remove impurities.

### Stage IV Separation

The nicotine is eluted from the cation exchange resin column by 1M sulphuric acid. The Nicotine salt is precipitated and collected from the bottom of the vessel.

### Stage V Filtration and purification

The Nicotine salt is filtrated and purified by passing it through a stainless steel super centrifuge (8000 rpm for at least 6 minutes) to remove traces of water.

### Stage VI Testing

After the filtration and purification of Nicotine Alkaloid, it was then tested for nitrosamine content (TSNA, i. e. NNN, NNK, NAT, and NAB). The result is shown in the table below batch 001 T. The total content, when tested, was at least less than 0.1 ug/g dry tobacco.
Thus, as indicated, the total level of nitrosamines in the tobacco can be selectively reduced.

### Stage VII Packing

The purified nicotine is packed in epoxycoated drums under an inert gas such as nitrogen and stored in cool and dry place.

### EXAMPLE 2 (not according to the invention)

### EXTRACTION AND PURIFICATION OF NICOTINE FROM FRESH LEAVES USING AN ORGANIC SOLVENT AND SUBSEQUENT SEPARATION USING A CATION EXCHANGE RESIN.

### Stage 1 Mixing

Fresh tobacco leaves from tobacco plants (="green tobacco") and calcium carbonate (CaCO3, limestone) or calcium hydroxide (slaked lime) 10 % by weight are mixed in a Mixing machine (ribbon) of Machine Steel. Water is added to bring the moisture of the mixture to 25 % humidity.

### Stage II Percolation

The slightly humid raw tobacco mixture is packed into a Machine Steel percolator, i.e. a cylindrical extractor. Water is percolated through the mixture to get a nicotinized broth.

### Stage III Extraction

This nicotinized broth, i.e. the nicotine containing aqueous mother-liquor is subjected to liquid - liquid extraction with a water-immiscible non-polar solvent in tall columns of Machine Steel or Stainless Steel for liquid-liquid extraction. Several solvents have been used including ethyl-acetate, hexane, cyclohexane and heptane. Nicotine is thereby transferred to the organic solvent. The liquor leaving the column by the bottom is essentially free of nicotine alkaloid. Liquid-liquid extraction not only separates and isolates the alkaloid rapidly, but it also contributes to its purification, since some impurities are insoluble in the organic solvent.

### Stage IV Separation

The nicotine concentration in an organic water-immiscible non-polar solvent is estimated by titration. Ten (10) ml of the solvent is mixed with 10 ml of 10normal H2SO4 in a beaker and titrated against 10normal NaOH solution using Phenolpthalin as indicator. The count will determine the quantity of Nicotine in the solvent. The greater the count the greater the quantity of Nicotine dissolved in the solvent and a calculated quantity of dilute aqueous Sulphuric acid is added under agitation in a Reactor vessel Machine Steel or Stainless Steel to form Nicotine Sulphate 40 %. Nicotine Sulphate 40 % being insoluble and heavier than the organic water-immiscible non-polar solvent is collected from the bottom of the vessel.

The Nicotine Sulphate 40 % is subsequently dissolved in purified water and transferred to a moderate-strong acid cation exchange resin, i.e., Amberlite™ IPR64 (Rohm and Haas/Ion Exchange Resins, Philadephia, PA, USA) or Indion 464 supplied by Ion Exchange (India) Ltd. packed in tall columns of Machine Steel or Stainless Steel. The nicotine - nicotine cation exchange resin complex is washed with purified water to remove impurities. The nicotine is eluted from the cation exchange resin column by 1M sulphuric acid. The Nicotine salt is precipitated and collected from the bottom of the vessel.

### Stage V Filtration and purification

The Nicotine salt is filtrated and purified by passing it through a stainless steel super centrifuge (8000 rpm for at least 6 minutes) to remove traces of water.

### Stage VI Packing

The purified Nicotine Sulphate is packed in epoxycoated drums and stored in cool and dry place.

### Stage VII Alkalization

Nicotine sulphate 40 % is taken into a Stainless Steel reactor and Sodium Hydroxide is added under slow agitation. After alkalization is complete, Nicotine 95 % separates out on top and is decanted.

### Stage VIII Purification of Nicotine Alkaloid

Nicotine Allcaloid is washed again with strong alkali (NaOH) water solution and separated by Super centrifugation to give pure Nicotine Alkaloid 98 %.

### Stage IX Distillation of Nicotine Alkaloid

Nicotine Alkaloid of 97 % to 99,9 % purity is taken in the distillation equipment (preferably of glass or glass lined). Under high vacuum (almost 760mm), the impurity (water) starts distilling at approx 70 degrees centigrade. After the distilling slows down (after approx 4 % of the quantity taken), the receiver flask is emptied (preferable changed) for the collection of Nicotine Allcaloid. With the vacuum kept high, Nicotine starts distilling at approx 140-160 degrees centigrade. The distilled alkaloid is then stored in either glass or stainless steel vessel under inert atmosphere.

### Stage X Testing.

After the distillation of Nicotine Alkaloid, it was then tested for nitrosamine content (TSNA, i. e. NNN, NNK, NAT, and NAB). The result is shown in the table below batch 002 T. The total content, when tested, was at least less than 0.1 ug/g dry tobacco.

Thus, as indicated, the total level of nitrosamines in the tobacco can be selectively reduced.

### Stage XI Packing

The purified nicotine is packed in epoxycoated drums under an inert gas such as nitrogen and stored in cool and dry place.

### EXAMPLE 3

### EXTRACTION AND PURIFICATION OF NICOTINE FROM DEEP FROZEN LEAVES USING AN ORGANIC SOLVENT, CATION EXCHANGE RESIN AND SUBSEQUENT DISTILLATION.

### Stage 1 Mixing

Tobacco leaves from tobacco plants (="green tobacco") are collected and deep frozen (-20°C) within less than 3 hours.

### Stage II Mixing and Percolation

The deep frozen tobacco leaves and calcium carbonate (CaCO3, limestone) or calcium hydroxide (slaked lime) 10 % by weight are mixed in a Mixing machine (ribbon) of Machine Steel Water is added to bring the moisture of the mixture to 25 % humidity. The slightly humid raw tobacco mixture is packed into a Machine Steel percolator, i.e. a cylindrical extractor. Purified water is percolated through the mixture to get a nicotinized broth.

### Stage III Extraction

This nicotinized broth, i.e. the nicotine containing aqueous mother-liquor is subjected to liquid - liquid extraction with an organic water-immiscible non-polar solvent, i.e., ethyl-acetate or another solvent as mentioned above, in tall columns of Machine Steel or Stainless Steel for liquid-liquid Extraction. Nicotine is thereby transferred to the organic solvent. The mother-liquor leaving the column by the bottom is essentially free of nicotine alkaloid. Liquid-liquid extraction not only separates and isolates the alkaloid rapidly, but it also contributes to its purification, since some impurities are insoluble in the organic solvent.

### Stage IV Separation

The nicotine concentration in the organic solvent is estimated by titration and a calculated quantity of dilute aqueous Sulphuric acid is added under agitation in a Reactor vessel Machine Steel or stainless steel to form Nicotine Sulphate 40 %. Nicotine Sulphate 40 % being insoluble and heavier than the organic solvent, is collected from the bottom of the vessel.

The Nicotine Sulphate 40 % is subsequently dissolved in purified water and transferred to a moderate-strong acid cation exchange resin i.e., Amberlite™ IPR64 (Rohm and Haas/Ion Exchange Resins, Philadephia, PA, USA) packed in tall columns of Machine Steel or Stainless Steel. The nicotine - nicotine cation exchange resin complex is washed with purified water to remove impurities. The nicotine is eluted from the cation exchange resin column by 1M sulphuric acid. The Nicotine-salt is precipitated and collected from the bottom of the vessel.

### Stage V Filtration and purification

The Nicotine salt is filtrated and purified by passing it through a stainless steel super centrifuge (8000 rpm for at least 6 minutes) to remove traces of water.

### Stage VI Packing

The purified Nicotine Sulphate is packed in epoxycoated drums and stored in cool and dry place.

### Stage VII Alkalization

The purified Nicotine Sulphate is taken into a Stainless Steel reactor and Sodium Hydroxide is added under slow agitation. After alkalization is complete, Nicotine 95 % separates out on top and is decanted.

### Stage VIII Purification of Nicotine Alkaloid

Nicotine Alkaloid is washed again with strong alkali (NaOH) water solution and separated by Super centrifugation to give pure Nicotine Alkaloid 98 %.

### Stage IX Distillation of Nicotine Alkaloid

Nicotine Alkaloid of 97% to 99,9% purity is taken in the distillation equipment (preferably of glass or glass lined). Under high vacuum (almost 760mm), the impurity (water) starts distilling at approx 70 degrees Centigrade. After the distilling slows down (after approx 4% of the quantity taken), the receiver flask is emptied (preferable changed) for the collection of Nicotine Alkaloid. With the vacuum kept high, Nicotine starts distilling at approx 140-160 degrees centigrade. The distilled alkaloid is then stored in either glass or stainless steel vessel under inert atmosphere.

### Stage X Testing.

After the distillation of Nicotine Alkaloid, it was then tested for nitrosamine content (TSNA, i. e. NNN, NNK, NAT, and NAB). The result is shown in the table below batch 003 T. The total content, when tested, was at least less than 0.1 ug/g dry tobacco.

Thus, as indicated, the total level of nitrosamines in the tobacco can be selectively reduced.

### Stage XI Packing

The purified nicotine is packed in epoxycoated drums under an inert gas such as nitrogen and stored in cool and dry place.

### EXAMPLE 4 (not according to the invention)

### EXTRACTION AND PURIFICATION OF NICOTINE FROM TOBACCO DUST USING AN ORGANIC WATER-IMMISCIBLE NON-POLAR SOLVENT, CATION EXCHANGE RESIN AND SUBSEQUENT DISTILLATION.

### Stage 1 Mixing

Tobacco dust (stalks, top leaves, stem, scraps and dusts are tobacco wastes) and calcium carbonate (CaCO3, limestone) or calcium hydroxide (slaked lime) 10 % by weight are mixed in a Mixing machine (ribbon type) of Machine Steel. Water is added to bring the moisture of the mixture to 25 % humidity.

### Stage II Percolation

The slightly humid raw tobacco mixture is packed into a Machine Steel percolator, i.e. a cylindrical extractor. Water is percolated through the mixture to get a nicotinized broth.

### Stage III Extraction

This nicotinized broth, i.e. the nicotine containing aqueous mother-liquor is subjected to liquid - liquid extraction with an organic water-immiscible non-polar solvent, ethyl acetate, in tall columns of Machine Steel or Stainless Steel for liquid-liquid extraction. Nicotine is thereby transferred to an organic water-immiscible non-polar solvent. The liquor leaving the column by the bottom is essentially free of nicotine alkaloid. Liquid-liquid extraction not only separates and isolates the alkaloid rapidly, but it also contributes to its purification, since some impurities are insoluble in the organic solvent.

### Stage IV Separation

The nicotine concentration in an organic water-immiscible non-polar solvent is estimated by titration. Ten (10)ml of the solvent is mixed with 10 ml of 10normal H2SO4 in a beaker and titrated against 10normal NaOH solution using Phenolpthalin as indicator. The count will determine the quantity of Nicotine in the solvent. The greater the count the greater the quantity of Nicotine dissolved in the solvent and a calculated quantity of dilute aqueous Sulphuric acid is added under agitation in a Reactor vessel Machine Steel or Stainless Steel to form Nicotine Sulphate 40 %. Nicotine Sulphate 40 % being insoluble and heavier than the organic water-immiscible non-polar solvent is collected from the bottom of the vessel.

The Nicotine Sulphate 40 % is subsequently dissolved in purified water and transferred to a moderate-strong acid cation exchange resin i.e., Amberlite™ IPR64 (Rohm and Haas/Ion Exchange Resins, Philadephia, PA, USA) or Indion 464 supplied by Ion Exchange (India) Ltd. packed in tall columns of Machine Steel or Stainless Steel. The nicotine - nicotine cation exchange resin complex is washed with purified water to remove impurities. The nicotine is eluted from the cation exchange resin column by 1M sulphuric acid. The Nicotine salt, 95%, is precipitated and collected from the bottom of the vessel.

### Stage V Filtration and purification

Nicotine Sulphate 95 % is filtrated and purified by passing it through a Stainless Steel super centrifuge (8000 rpm for at least 6 minutes) to separate suspended particles and remove traces of the organic water-immiscible non-polar solvent.

### Stage VI Packing

The purified Nicotine Sulphate is packed in epoxycoated drums under an inert gas such as nitrogen and stored in cool and dry place.

### Stage VII Alkalization

The purified Nicotine Sulphate is taken into a Stainless Steel reactor and Sodium Hydroxide is added under slow agitation. After alkalization is complete, Nicotine 95-97 % separates out on top and is decanted.

### Stage VIII Purification of Nicotine Alkaloid

Nicotine Alkaloid is washed again with strong alkali (NaOH) water solution and separated by Super centrifugation to give pure Nicotine Alkaloid 97-98 %.

### Stage IX Distillation of Nicotine Alkaloid

Nicotine Alkaloid of 97 % to 99,9 % purity is taken in the distillation equipment (preferably of glass or glass lined). Under high vacuum (almost 760mm), the impurity (water) starts distilling at approx 70 degrees Centigrade. After the distilling slows down (after approx 4% of the quantity taken), the receiver flask is emptied (preferable changed) for the collection of Nicotine Alkaloid. With the vacuum kept high, Nicotine starts distilling at approx 140-160 degrees centigrade. The distilled alkaloid is then stored in either glass or stainless steel vessel under inert atmosphere.

### Stage X Testing.

After the distillation of Nicotine Alkaloid, it was then tested for nitrosamine content (TSNA, i. e. NNN, NNK, NAT, and NAB). The result is shown in the table below batch 004 T. The total content, when tested, was at least less than 0.1 ug/g dry tobacco. Thus, as indicated, the total level of nitrosamines in the tobacco can be selectively reduced.

### Stage XI Packing

The purified nicotine is packed in epoxycoated drums under an inert gas such as nitrogen and stored in cool and dry place.

### EXAMPLE 5

### REFINED TOBACCO CONTAINING PURIFIED NICOTINE

### Stage I Washings of the insoluble fraction

After extraction of tobacco dust (stalks, top leaves, stem, scraps and dusts are tobacco wastes), fresh or deep frozen tobacco leaves according to the examples 1-4 above the insoluble fraction is refined by washing 5 times with pure water (acidic, alkaline and neutral) and finally with ethyl alcohol for 30 minutes each.

### Stage II Centrifugation and drying of insoluble fraction

The insoluble fraction is centrifuged for at least 6 minutes at 8000 revolutions per minute to remove the major part of the water.

### Stage III Centrifugation and drying of insoluble fraction

After centrifugation the refined fibers are dried by Fluidized Bed Drying until having less than 1% humidity.

### Stage IV Reconstitution of the tobacco by impregnation

After centrifugation and Fluidized Bed Drying the refined fibers (the web), are impregnated with the high purity nicotine solution according to examples 1 -4 so that a purified finished tobacco is obtained that has nicotine content of 0.5-10% by weight.

### Stage V Testing of the tobacco

After the purification and impregnation the tobacco was then tested for nitrosamine content (TSNA, i. e. NNN, NNK, NAT, and NAB). The total content, was at least less than 0.1 ug/g dry tobacco. Thus, as indicated, the total level of nitrosamines in the tobacco can be selectively reduced without substantially reducing other levels of components in the tobacco, such as carbohydrates, starch and cellulose. See table 1.

### Stage VI Packing

The tobacco product may be formulated into one or more preparations, such as sachets, pouches, stick-pack, buccal pads or patches or compressed into chewing tablets, lozenges, resoriblettes or tablets for sucking. The resulting product is the packed in air tight packages to prevent losses by oxidization and/or vaporization of the nicotine. During storage pending packaging in consumer packs the purified tobacco as well as the formulated products derived from it are packed in epoxycoated drums under an inert gas such as nitrogen and stored in cool and dry place.

**Table1. Total content of some impurities in different batches of tobacco produced by the different method in the examples above as well as some natural sources. Tobacco 1-5 represent natural sources.**

| | µg/g | | | | | | |
|---|---|---|---|---|---|---|---|
| | Sum TSNA | NNN | NNK | NAB | NAT | Nicotine assay | TSNA (ug) / nicotine (mg) |
| Batch Nr | | | | | | | |
| Tobacco 1^{a} | 21,590 | | | | | 2,1 | 10,281 |
| Tobacco 2 ^{a} | 19,550 | | | | | 1,2 | 16,292 |
| Tobacco 3 ^{a} | 14,539 | | | | | 2,6 | 5,592 |
| Tobacco 4 ^{a} | 54,590 | | | | | 2,9 | 18,824 |
| Tobacco 5 ^{a} | 6,590 | | | | | 1,4 | 4,707 |
| 001T | <0,01 | <0,01 | <0,01 | <0,01 | <0,01 | 2 | 0,005 |
| 002T | <0,01 | <0,01 | <0,01 | <0,01 | <0,01 | 2 | 0,005 |
| 003T | <0,01 | <0,01 | <0,01 | <0,01 | <0,01 | 2 | 0,005 |
| 004T | <0,01 | <0,01 | <0,01 | <0,01 | <0,01 | 2 | 0,005 |
| 008 ^{b} | 0,059 | 0,030 | 0,000 | 0,000 | 0,030 | 3 | 0,01 |
| 009 ^{b} | 0,117 | 0,06 | 0,02 | <0.01 | 0,04 | | |
| 017 ^{b} | 0,08 | 0,03 | 0,03 | <0,01 | 0,03 | 5 | 0,016 |
| 018 A ^{b} | 0,110 | 0,02 | 0,02 | <0,01 | 0,02 | 0,1 | 0,600 |
| 018 ^{b} | 0,060 | 0,04 | 0,05 | <0,01 | 0,02 | 5 | 0,022 |
| GT ^{c} | <0,01 | <0,01 | <0,01 | <0,01 | <0,01 | 2 | 0,005 |
| GT6 ^{c} | 0,040 | 0,02 | 0,01 | <0,01 | 0,01 | 2 | 0,020 |
| GTF1 ^{c} | <0,01 | <0,01 | <0,01 | <0,01 | <0,01 | 2 | 0,005 |
| GTF2 ^{c} | <0,01 | <0,01 | <0,01 | <0,01 | <0,01 | 2 | 0,005 |
| GTF3 ^{c} | <0,01 | <0,01 | <0,01 | <0,01 | <0,01 | 3 | 0,003 |
| GTF5 ^{c} | <0,01 | <0,01 | <0,01 | <0,01 | <0,01 | 5 | 0,002 |

| | | | | | | | |
|---|---|---|---|---|---|---|---|
| a/ Tobacco purchased from different suppliers. b/ Results from farmed, harvested, treated and/or processed. c/ Results from farmed, harvested and treated. 018 were tested prior to purification (A) and after purification. | | | | | | | |

### EXAMPLE 6

### NICOTINE CHEWING GUM PRODUCT

To obtain 1000 g of chewing gum 22 g nicotine obtained in the examples 1-4 was mixed with chewing gum premix, consisting of 600 g Dreyco Gum Base (L. A. Dreyfus Company, 3775 Park Avenue, Edison, New Jersey, USA), or Nicol 8 (Gum Base Co, Via Nerviano, 25, 20020 Lainate, Milan, Italy), 205 g sorbitol, 20g Xylitol, 55 g talc, 40 g sodium bicarbonate, 20 g sodium carbonate 3 g aspartame, 1 g Acesulfame Potassium, 5 g menthol and flavouring oils. Mixing of nicotine, flavouring, buffer and premelted gum base was made in a gum kneader.

After the following steps of manufacture nicotine chewing gum was obtained: Extrusion, Cooling, Rolling, Scoring, Hardening and Wrapping.

The following values for strength and purity were achieved:

### Testing of the finished gum product

### Nicotine assay

| | |
|---|---|
| Nicotine assay (initial)* | 2.00-2.20 mg/piece |
| Nicotine assay (after 6 months storage) * | 1.8 - 2.20 mg/piece |

### Impurities/Degradation products

| | | |
|---|---|---|
| Cotinine | ≤ 0.5 %** | ≤ 0.75 %*** |
| Nicotine trans - N - oxide | ≤ 0.5 %** | ≤ 2.5 %*** |
| Nicotine cis - N - oxide | ≤ 0.5 %** | ≤ 1.75 %*** |
| Total | ≤ 1.0 %** | ≤ 5 %*** |
| | | |
| * = | Determined on finished product. | |
| ** = | Values on nicotine carrier used in the batch and expressed as percent of its nicotine assay value. | |
| *** = | Determined on finished product and expressed as percent of its nicotine assay value. | |

| | | |
|---|---|---|
| Note: Carrier being polacrilex, cyclodextrin, starch, cellulose and cellulose derivatives, chewing gum bases, gelatin, alginate, PVP and tobacco and other fibers. | | |

The gum was then tested for nitrosamine content (TSNA, i. e. NNN, NNK, NAT, and NAB). The total content was at least less than 0.1 ug nitrosamines/g dry tobacco.

### EXAMPLE 7

### A SNUFF (SNUS) TOBACCO PRODUCT

The tobacco from example 5 is prepared into portion snuff (snus) pouches (sachets) of woven or non-woven material. Snuff (snus) was manufactured in different strengths e.g. 1, 3, and 6 mg per pouches. The nicotine amounts of tobacco were different in the formulations and were varied due to assay of the tobacco batches. Typically tobacco was mixed with excipients as fillers (dextrin e.g. polydextrin, maltodextrin, natural fibres, cellulose, starches or other suitable fillers), sweeteners (Xylitol, sorbitol, maltitol, mannitol, saccharin, sodium saccharin, cyclamates, Acesulfan K), adjutants to increase floe properties (Avicel; Microcrystalline cellulose, talc) and natural and nature identical aromas and flavours. A typical formulation for 3 mg snus portion, Tobacco 10 to 24 %, Fibre 10 to 20 %, Polydextrose 5 to 15 %, Sodium Chloride 8 %, Sodium bicarbonate 7 %, polyvinyl pyrrolidon 3 %, Talc 2 %, Avicel 5 to 15 %, flavours quantum sati.

The tobacco product, such as sachet, pouch, stick-pack, is packed in air tight packages to prevent losses by oxidization and/or vaporization of the nicotine.

### Example 8

### A TOBACCO CHEWING GUM PRODUCT

A tobacco chewing gum (1000g) was manufactured by mixing a premix of 15 g glycerine and 15 g finely grinded tobacco (example 5) with the chewing gum premix in example 8, consisting of 600 g Dreyco Gum Base (L. A. Dreyfus Company, 3775 Park Avenue, Edison, New Jersey, USA), or Nicol 8 (Gum Base Co, Via Nerviano, 25, 20020 Lainate, Milan, Italy), 205 g sorbitol, 20g Xylitol, 55 g talk, 50 g sodium bicarbonate, 3 g aspartame, 1 g Acesulfame Potassium, 5 g menthol and flavouring oils.

Mixing of tobacco, flavouring, buffer and premelted gum base was made in a gum kneader.

After the following steps of manufacture a tobacco chewing gum was obtained: Extrusion, Cooling, Rolling, Scoring, Hardening And Wrapping.

### Tobacco chewing gum:

| | |
|---|---|
| Nicotine assay (initial) | 2.00-2.20 mg/piece |
| (after 6 months storage) | 1.8 - 2.20 mg/piece |

The tobacco chewing gum was then tested for nitrosamine content (TSNA, i. e. NNN, NNK, NAT, and NAB). Nitrosamines were assayed by a gas chromatograph system, LC-MS-MS. The total content was at least less than 0.1 ug nitrosamines/g dry tobacco.

### Example 9

### ANALYSIS OF THE NITROSAMINES

Nitrosamines were assayed the method published in J Chromatogr A. 2003 Aug 8; 1008(2):135-43, Jansson et al, Analysis of tobacco-specific N-nitrosamines in snuff by ethyl acetate extraction and liquid chromatography-tandem mass spectrometry. A rapid, selective and sensitive method for routine analysis of the four tobacco-specific N-nitrosamines, N'-nitroso-nornicotine, N'-nitrosoanatabine, N'-nitrosoanabasine and 4-(methyl-nitrosamino)-1-(3-pyridyl)-1-butanone in snuff has been developed. The nitrosamines were isolated by ethyl acetate extraction and analysed by LC-MS-MS. Except for evaporation and filtration, no additional clean-up steps are needed in the proposed method. The detection limits for standard in solvent are between 0.0005 and 0.001 microg/ml (0.005 and 0.01 microg/g).

### Example 10

### MEASURMENT OF COLOUR INDEX

Colour index was measured on the supernatant after dispersing and incubating the tobacco for 30 minutes followed by filtration. Measurements were made according to a modified European Pharmacopea method.
As a measure of the colour of nicotine product and /or tobacco thereof, the Visual Color Index- VCI is introduced.

The pouches containing the refined tobacco product was placed in water in a testing tube made of clear glass with or with out stirrer. The Visual Colour Index is regarded as follows

| | **VCI** | | **VCI** | | **VCI** |
|---|---|---|---|---|---|
| Clear solution | **1** | No opalescent | **1** | No particles | **1** |
| Slightly light brownish solution | **2** | Tendency opalescent | **2** | Some particles | **2** |
| Light brownish solution | **3** | Slightly opalescent | **3** | Particles | **3** |
| Brownish solution | **4** | Opalescent | **4** | Particles that gives tendency to sediment | **4** |
| Dark brownish solution | **5** | White opalescent | **5** | Particles that gives sediment | **5** |
| Deep dark brownish solution | **6** | White opalescent unclear | **6** | Particles that gives clear sediment | **6** |

The Visual Colour Index has been tested for the refined tobacco and common Swedish Snus purchased in any ordinary shops, gives the following VCI Index when tested;

### Results; The sum at each time point is summarised;

| **min.** | **The invented Tobacco** | **V C I** | **Common "snus" brands** | **V CI** |
|---|---|---|---|---|
| 0 | Clear solution, No opalescence, No particles | **3** | Clear solution, No opalescence, No particles | **3** |
| 1 | Clear solution, No opalescence, No particles | **3** | Slightly light brownish solution, No opalescence, Some particles | **4** |
| 2 | Clear solution, No opalescence, No particles | **3** | Light brownish solution, No opalescence, Some particles | **5** |
| 5 | Clear solution, Tendency opalescenet, No particles | **3** | Brownish solution, No opalescence, Particles | **8** |
| 10 | Clear solution, Tendency opalescent, No particles | **3** | Dark brownish solution, No opalescence, Particles that gives sediment | **11** |
| 20 | Clear solution, Tendency opalescent, No particles | **3** | Deep dark brownish solution, Opalescence, Particles that gives sediment | **16** |

The colour Index gives a product that has less risk for colouring the teeth and less smell from the tobacco.

## Claims

1. A method of producing a tobacco product comprising the steps of;
a. providing tobacco material comprising nicotine and fibres, wherein the tobacco plant material is lamina, which is harvested and directly transferred into a temperature being below 0°C,
b. separating said fibres from said tobacco material,
c. extracting nicotine from said tobacco material,
d. obtaining nicotine, wherein said nicotine comprises less than 0.1 ug nitrosamines/mg nicotine.

2. The method according to claim 1, wherein the tobacco material in step a) is green tobacco plant material.

3. The method according to claim 1, wherein said tobacco material is frozen in liquid nitrogen.

4. The method according to any of claims 1-3, wherein said extraction in step c) is performed by the use of a cationic exchange resin.

## Patentansprüche

1. Verfahren für die Herstellung eines Tabakerzeugnisses, umfassend die folgenden Schritte;
a. Bereitstellen eines Tabakmaterials, umfassend Nikotin und Fasern, wobei das Tabakpflanzenmaterial Lamina ist, die geerntet und direkt in eine Temperatur, die unter 0° C liegt, überführt wird,
b. Trennen der Fasern vom Tabakmaterial,
c. Extraktion des Nikotins vom Tabakmaterial,
d. Erhalten des Nikotins, wobei das Nikotin weniger als 0,1 ug Nitrosaminen/mg Nikotin umfasst.

2. Verfahren nach Anspruch 1, wobei das Tabakmaterial im Schritt a) ein grünes Tabakpflanzenmaterial ist.

3. Verfahren nach Anspruch 1, wobei das Tabakmaterial in flüssigem Stickstoff gefroren wird.

4. Verfahren nach einem der Ansprüche 1-3, wobei die Extraktion im Schritt c) durch die Verwendung eines kationischen Austauschharzes durchgeführt wird.

## Revendications

1. Procédé pour la fabrication d'un produit du tabac comprenant les étapes de:
a. fournir de la matière de tabac comprenant de la nicotine et des fibres, la matière de la plante de tabac étant la lamina qui est récoltée et transférée directement dans une température étant inférieure à 0°C,
b. séparer lesdites fibres de ladite matière de tabac,
c. extraire de la nicotine de ladite matière de tabac,
d. obtenir de la nicotine, ladite nicotine comprenant moins de 0,1 ug nitrosamines/mg nicotine.

2. Procédé selon la revendication 1, dans lequel la matière de tabac dans l'étape a) est une matière de plante de tabac vert.

3. Procédé selon la revendication 1, dans lequel ladite matière de tabac est gelée dans l'azote liquide.

4. Procédé selon l'une quelconque des revendications 1 à 3, dans lequel ladite extraction c) est réalisée par l'utilisation d'une résine échangeuse de cations.
